(19) 

Europäisches
Patentamt
European
Patent Office
Office européen
des brevets

(11) **EP 4 502 139 A1**

(12) **EUROPEAN PATENT APPLICATION**
published in accordance with Art. 153(4) EPC

(43) Date of publication:
**05.02.2025 Bulletin 2025/06**

(21) Application number: 23810583.7

(22) Date of filing: **03.03.2023**

(51) International Patent Classification (IPC):
*C12N 1/18* (2006.01)      *A21D 8/04* (2006.01)
*C12R 1/865* (2006.01)

(52) Cooperative Patent Classification (CPC):
**A21D 8/04; A21D 13/00; C12N 1/16; C12N 1/18;**
C12R 2001/865

(86) International application number:
**PCT/CN2023/079575**

(87) International publication number:
**WO 2023/226507 (30.11.2023 Gazette 2023/48)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 27.05.2022  CN 202210592898

(71) Applicant: Angel Yeast Co., Ltd
**Yichang, Hubei 443003 (CN)**

(72) Inventors:
• **QIN, Xianwu
Yichang, Hubei 443003 (CN)**

• **SUN, Yafang
Yichang, Hubei 443003 (CN)**
• **GUO, Tianfen
Yichang, Hubei 443003 (CN)**
• **SHI, Yu
Yichang, Hubei 443003 (CN)**
• **WANG, Long
Yichang, Hubei 443003 (CN)**
• **KUANG, Jinbao
Yichang, Hubei 443003 (CN)**

(74) Representative: **karo IP
Patentanwälte PartG mbB
Steinstraße 16-18
40212 Düsseldorf (DE)**

(54) **HIGH-SUGAR-PERMEATION-RESISTANT SACCHAROMYCES CEREVISIAE STRAIN AND USE**

(57)    The present invention provides a high-sugar-permeation-resistant saccharomyces cerevisiae strain and use. The saccharomyces cerevisiae strain provided in the present invention is saccharomyces cerevisiae AMCC 31195 strain, which is preserved in the China Center for Type Culture Collection (CCTCC) with an deposit number of CCTCC NO: M 20211685. The saccharomyces cerevisiae AMCC 31195 strain provided in the present invention has the characteristics of high sugar permeation pressure resistance, cold permeation shock resistance and organic acid resistance.

EP 4 502 139 A1

## Description

### Technical Field

The present invention belongs to the field of microorganisms and specifically relates to a high-sugar-permeation-resistant brewing high-sugar-resistant, cold permeation shock-resistant, and organic acid-resistant yeast strain and use thereof.

### Background Art

Fermented flour products, such as steamed bread, steamed buns, breads, and the like, account for a large proportion of people's dietary structure as staple food. Yeast is an essential raw material in the production of fermented pasta, and different yeasts affect the quality and taste of fermented pasta. With the improvement of people's living standards, there are more and more demands on the quality and taste of fermented flour products, such as extending shelf life and high sugar content.

### Summary of the Invention

In the process of making bread, more sugar is often added, and the fermentation performance of yeast decreases sharply in the high-sugar environment.

In the bread-making process, the temperature of the environment is often too high, which causes yeast to rise too quickly, resulting in the decline of product quality. In order to reduce the temperature of the dough, low-temperature water is usually added to reduce the temperature, but the yeast cells will enter into shock state when they encounter a low-temperature environment, thus affecting the normal growth of yeast cells. Therefore, there is an urgent demand for the cold permeation shock tolerance of yeast.

To extend the shelf life of bread, preservatives are often added to bread to extend shelf life. Preservatives commonly used in bread processing are typically organic acids and their salts, such as propionic acid, acetic acid, calcium propionate, sodium propionate, and sodium acetate. These organic acids and their salts have some side effects on the bread fermentation process, which will affect the bread dough fermentation.

It can be seen therefrom that in order to meet the practical application scenarios of bakery products and the need for preservation, a high-sugar permeation pressure resistant, cold permeation shock-resistant, and organic acid-resistant yeast strain is urgently needed.

The present invention provides a saccharomyces cerevisiae strain, which is saccharomyces cerevisiae AMCC 31195 strain, which is deposited in the China Center for Type Culture Collection (CCTCC) with deposit number CCTCC NO: M 20211685.

The present invention also provides a fermentation preparation method for a Saccharomyces cerevisiae microbial inoculum, the method includes the step of culturing the Saccharomyces cerevisiae strain.

Preferably, the preparation method includes the steps of:

(1) amplifying and culturing the Saccharomyces cerevisiae strain;
(2) adding the product obtained in step (1) to a liquid medium, and fermenting and culturing at 26-32°C.

The present invention also provides a microbial inoculum including the Saccharomyces cerevisiae AMCC 31195 strain.

Preferably, the microbial inoculum is obtained by the fermentation preparation method.

The present invention also provides the use of the Saccharomyces cerevisiae strain or the microbial inoculum in fermentation.

The present invention also provides the use of the Saccharomyces cerevisiae strain or the microbial inoculum in a dough.

The present invention also provides a dough including the Saccharomyces cerevisiae strain or the microbial inoculum.

Preferably, the dough includes flour and the Saccharomyces cerevisiae strain in a mass ratio of 100:0.5-5.

Preferably, the dough includes flour and sugar in a mass ratio of 100:0-40; Preferably, the dough includes flour and sugar in a mass ratio of 100:15-40;

Preferably, the sugar is sucrose.

Preferably, the dough further includes a preservative;

preferably, the preservative is an organic acid and salts thereof;

preferably, the organic acid is one or two of propionic acid and acetic acid, and the organic acid salt is one or a combination of two or more of calcium propionate, sodium propionate, and sodium acetate;

preferably, the mass ratio of flour to preservative in the dough is 100:0-1, preferably 100:0-0.6. The present invention also provides a preparation method for the dough including the steps of kneading the dough with water at -0-35°C.

The water at 0°C in the present invention may be ice at 0°C or a mixture of ice and water at 0°C or liquid water at 0°C.

In the preparation of the dough according to the present invention, the water, the flour, and the saccharomyces cerevisiae may be added in any order, such as mixing the flour and the saccharomyces cerevisiae and then adding water at 0-35°C to the flour, adding the flour to water at 0-35°C and then adding the saccharomyces cerevisiae, or adding the saccharomyces cerevisiae to water at 0-35°C and then adding the flour.

The present invention also provides a dough product prepared from the dough.

Preferably, the dough product is bread.

The saccharomyces cerevisiae AMCC 31195 strain provided in the present invention has the characteristics of high-sugar permeation pressure resistance, cold permeation shock resistance, and organic acid resistance.

## Strain deposit information

The strain Saccharomyces cerevisiae AMCC 31195 of the present invention was deposited in the China Center for Type Culture Collection (CCTCC) on December 29, 2021, with deposit number CCTCC NO: 20211685, deposited at Wuhan University, Wuhan, China, postal code: 430072, Tel: (027)-68754052.

The strain Saccharomyces cerevisiae AMCC 30002 of the present invention was deposited in the China Center for Type Culture Collection (CCTCC) on March 29, 2022, with deposit number CCTCC NO: M 2022338, deposited at Wuhan University, Wuhan, China, postal code: 430072, Tel: (027)-68754052.

The strain Saccharomyces cerevisiae AMCC 30004 of the present invention was deposited in China Center for Type Culture Collection (CCTCC) on March 29, 2022, with deposit number CCTCC NO: 2022339, deposited at Wuhan University, Wuhan, China, postal code: 430072, Tel: (027)-68754052.

## Detailed Description of the Invention

In the present invention, Saccharomyces cerevisiae strain AMCC 30002 and Saccharomyces cerevisiae strain AMCC 30004 are used as parent strains to hybridize to obtain Saccharomyces cerevisiae strain AMCC 31195 which is high-sugar permeation pressure resistant, cold permeation shock resistant and organic acid resistant.

A single spore of the parent strain was first prepared by using a yeast micromanipulator. After obtaining a generation of heterozygotes, a single spore is prepared, and the obtained single spore is hybridized with a single spore of the parent Saccharomyces cerevisiae strain AMCC 30004. After two rounds of hybridization, the obtained heterozygotes are verified and screened for traits. Then performing shake flask fermentation culture, and screening the biomass dry weight and fermentation activity indicators, wherein the biomass dry weight screening criterion is that the yeast milk biomass dry weight of the heterozygous strain reaches 90-100%, preferably 95-100% of that of any parent strain; the screening criterion of fermentation activity was that 1 h fermentation activity of heterozygous strain yeast milk with 16% sugar reached 90-100%, preferably 95-100% of that of any parent strain; 3 h fermentation activity with 16% + 1% calcium propionate is 85-100%, preferably 90-100% of that of any parent strain.

The heterozygous strains obtained from shake flask fermentation screening were cultured in a fermenter for a small test. The yeast obtained from the fermenter culture was used to prepare active dry yeast, the heterozygous strains without obvious abnormality in the preparation process of active dry yeast were screened. The obtained active dry yeast was used to prepare the dough containing 16% sugar + 0.6% calcium propionate, to screen heterozygous strains from which the active dry yeast prepared that had better fermentation performance in the environment of high sugar and organic acid. The screening criterion is that the 2 h fermentation activity of the heterozygous strain active dry yeast at 16% + 0.6% calcium propionate reached 100-120%, preferably 110-120% of that of any parent strain.

Finally, the heterozygous strains screened in the above steps were screened for cold permeation shock resistance. 20% sugar dough containing heterozygous strain active dry yeast was kneaded with crushed ice at 0°C and flour, and the leavening time of the dough was determined. The heterozygous strain with the shortest leavening time of the corresponding dough was used as the target strain to screen out the heterozygous strain with cold permeation shock resistance. Saccharomyces cerevisiae AMCC 31195 strain was obtained by screening, and the obtained Saccharomyces cerevisiae AMCC 31195 strain had high-sugar permeation pressure resistance, organic acid, and cold permeation shock resistance.

According to the present invention, a high-sugar wheat yeast strain with various industrial traits can obtain baker's yeast with multiple tolerance without the domestication process, which can solve the urgent market demand for high-sugar permeation pressure resistance, cold permeation shock resistance, and organic acid resistance and improve the competitiveness of leading products. The dough referred to in the present examples as x% sugar or x% calcium propionate means that the mass ratio of flour to sugar or flour to calcium propionate in the dough is 100:x.

Some sugar-free bread, soda biscuits, steamed bread, etc. are mainly made by sugar-free dough fermentation. Most of the flour is starch, which is converted into maltose by amylase in flour. Therefore, the ability of yeast to use maltose also determines the rising speed of sugar-free dough. The maltose-using enzymes of yeast include maltase and maltose permease. The yeast with such properties is called fast-fermenting yeast.

Sugar-tolerant yeast means that it has a higher tolerance to sucrose in sugar-containing doughs, i.e. the growth and fermentation properties in sugar-containing breads are higher than in normal yeasts.

The low-sugar resistant yeast is used in a dough system with about 7% sucrose, and the high-sugar resistant yeast is used in a dough system with a higher sucrose concentration, with a content of up to 25%. Sucrose cannot generally be directly used by microorganisms, while Saccharomyces cerevisiae contains a sucrose hydrolase capable of degrading sucrose, which acts on β-1,2 glycosidic bonds to hydrolyze sucrose into D-glucose and D-fructose, and then glucose and fructose enter the glycolytic pathway for use by the yeast, while the glucose and fructose generated due to the rapid decomposition of sucrose will increase the permeation pressure around the yeast cells. The yeast cell membrane is a selective semi-permeable biofilm. The activity of yeast cells is affected by the concentration of the external environment. When the cells are in a high permeation pressure environment, the water content and protoplast in the cells will leak out of the cell membrane to make the cells dehydrated and even die. Therefore, the high permeation environment that Saccharomyces cerevisiae faces in high-sugar dough affects its growth and fermentation performance. Therefore, the gas production capacity of sugar-free yeast is determined by maltose utilization enzyme activity, and the gas production capacity of sugar-tolerant yeast is determined by sucrase activity.

Reagents and instrument information used in the examples of the present invention are shown in Table 1 below.

Table 1

| Reagent/instrument | Model | Manufacturer |
|---|---|---|
| Yeast extract powder | | Angel yeast |
| Glucose | | SINOPHARM |
| Peptone | | Angel yeast |
| Agar | | HUIXING |
| Potassium acetate | | Hushi, SINOPHARM |
| 2 x PCR Mix | | TIANGEN |
| Analytical balance | ME4002E | METTLER TOLEDO |
| pH meter | PB-10 | Sartorius |
| Rapid water content meter | MJ33 | METTLER TOLEDO |
| Clean bench | SKJH-1109 | Shanghai Sukun |
| Constant temperature shaker | ZWYR-2102C | Shanghai Zhicheng |
| Biochemical incubator | SPX-158L | Ningbo Scientz |
| Constant temperature water bath | HH-2 | Jiangsu Guohua |
| PCR instrument | C1000 | BIO-RAD |
| Gel imaging system | GelDoc™XR + | BIO-RAD |
| Centrifuge | DL-5200B-II | Shanghai Feige |
| Electrophoresis apparatus | EPS-300 | Shanghai Tanon |
| Ultraviolet-visible light photometer | UV2310II | Hangzhou Allsheng |
| Optical microscope | CX43 | OLYMPUS |
| Full-automatic growth curve analyzer | BioscreenC | OY Growth Curves |
| Activity tester | SJA | SJA, Sweden |
| Electric heating blast drying box | DHG-9070A | Shanghai Jinghong |

The formula of the sporulation medium used in the examples of the present invention was: 1% potassium acetate, 0.1% yeast extract powder, 0.05% glucose, and 2% agar.

In the present example, each Saccharomyces cerevisiae strain was activated by using a YPD solid medium, and the formula of the YPD solid medium was: 1% yeast extract powder, 2% peptone, 2% glucose, and 2% agar.

In the examples of the present invention, each Saccharomyces cerevisiae strain was cultured in a YPD liquid medium, and the formula of the YPD solid medium was: 1% yeast extract powder, 2% peptone, and 2% glucose.

**Example I**

The Saccharomyces cerevisiae AMCC 30002 strain and Saccharomyces cerevisiae AMCC 30004 strain were used as parent strains for hybridization.

Saccharomyces cerevisiae AMCC30002 strain is a Saccharomyces cerevisiae strain bred by Angel Yeast Co., Ltd. which was collected from Yantai City, Shandong Province. Through the observation of an optical microscope, the colony of Saccharomyces cerevisiae strain was cheese-like in texture, milky white in color, smooth in surface and neat in edge, oval in microscopic shape and budding. After 26S rDNA gene identification, the strain was identified as Saccharomyces cerevisiae strain which had a food product attribute. It was deposited in the China Center for Type Culture Collection (CCTCC) on March 29, 2022, with deposit number CCTCC NO: M 2022338 (i.e. CCTCC M 2022338).

Saccharomyces cerevisiae AMCC30004 is a Saccharomyces cerevisiae strain bred by Angel Yeast Co., Ltd. which was collected from the Kazakh Autonomous Prefecture of Ili in Xinjiang Uygur Autonomous Region. Through the observation of an optical microscope, the colony of Saccharomyces cerevisiae strain was cheese-like in texture, milky white in color, smooth in surface and neat in edge, oval in microscopic shape and budding. After 26S rDNA gene identification, the strain was identified as Saccharomyces cerevisiae strain which had a food product attribute. It was deposited in the China Center for Type Culture Collection (CCTCC) on March 29, 2022, with deposit number CCTCC NO: M 2022339 (i.e. CCTCC M 2022339).

The two parent strains produce spores under the same conditions, a single spore was inoculated into a test tube of YPD liquid culture medium, shaken at 30°C, and cultured overnight, and then a hybridization combination was designed according to different matching, single spores of different matching were inoculated into a test tube of YPD liquid culture medium successively, shaken at 30°C, and cultured overnight, then diluted and spread onto a YPD plate, shaken at 30°C, and cultured overnight, and the growth of colonies was observed. The bigger colonies on the plate were selected and inoculated into the test tubes of YPD liquid medium, shaken at 30°C, and cultured overnight, the matching identification and sporulation tests were performed on them, and the heterozygote was selected. The resulting heterozygotes were inoculated into 100-well plates containing high sugar medium, ready for on-machine determination by Bioscreen C instrument, and the parameters were set as follows: the growth curve determination was performed after measuring data every 30 min at a temperature of 30°C for 48 h at a wavelength of 600 nm. The maximum specific growth rate is analyzed according to the growth curve results, and the specific formula is as follows:

$$\text{Maximum specific growth rate } (\mu) = (\ln OD2 - \ln OD1)/(t2 - t1)$$

OD1: The corresponding $OD_{600}$ value at t1;
OD2: The corresponding $OD_{600}$ value at t2;
t2: End time of logarithmic growth phase;
t1: Start time of logarithmic growth phase;

according to the analysis of Bioscreen C high-throughput data, the first three heterozygous strains with the maximum specific growth rate were selected as one-generation strong heterozygotes.

According to the above steps, a single spore was prepared again, and the obtained single spore was hybridized with the strain Saccharomyces cerevisiae AMCC 30004, heterozygotes screening and heterozygotes verification were performed, and the above steps were repeated to obtain heterozygotes of two rounds of hybridization, and the heterozygous strains with the first 100 maximum specific growth rate in the last round of heterozygotes were selected for a shake flask fermentation test.

The biomass was used as the screening index, and the heterozygous strains satisfying the biomass index among the 100 heterozygous strains obtained above were screened. The shake flask experiment was performed on the above-mentioned heterozygous strains, the strains were inoculated into a YPD liquid medium shake flask, shaken at 30°C, and cultured overnight, to obtain yeast milk after centrifugation and washing, water content detection was performed, and biomass dry weight was calculated, wherein the screening criterion is that the biomass dry weight of the heterozygous strains reaches 95-100% of that of any parent strain;

the following tests were performed sequentially on heterozygous strains with biomass dry weight advantage to screen heterozygous strains with high sugar resistance and organic acid resistance:

Test A: Detection of the 1 h fermentation activity of 280 g dough of 16% sugar dough system, the screening criterion was 95-100% of that of any parent strain; ,

Test B: Detection of the 3 h fermentation activity of 70 g dough of 16% sugar + 1% calcium propionate dough system, the screening criterion was 95-100% of that of any parent strain.

Table 2 shows the dough system formulas for Test A and Test B.

Table 2 Formulas of dough systems for Test A and Test B

| Test raw material | A(g) | B (g) |
|---|---|---|
| Flour | 00 | 100 |
| Sucrose | 16 | 16 |
| Salt | 1.4 | 1.4 |
| Dry yeast | 1 | 1 |
| Water | 45 | 45 |
| Calcium Propionate | - | 1 |

The heterozygous strains with high sugar resistance and organic acid resistance obtained by screening were subjected to subsequent screening.

The heterozygous strains with high sugar resistance and organic acid resistance were cultured in a fermenter for a small test. The yeast obtained from fermenter culture was used to prepare active dry yeast, the heterozygous strains without obvious abnormality in the preparation process of the dry yeast were selected. The obtained active dry yeast was used to prepare the dough containing 16% sugar + 0.6% calcium propionate, to screen heterozygous strains from which the active dry yeast prepared that had better fermentation performance in the environment of high sugar and organic acid. The screening criterion is that the 2 h fermentation activity of the heterozygous strain active dry yeast at 16% + 0.6% calcium propionate reached 100-120%, preferably 110-120% of that of any parent strain.

Finally, the cold permeation shock resistance screening was performed on the heterozygous strains from which the active dry yeast prepared that had better fermentation performance in the environment of high sugar and organic acid. 20% sugar dough containing heterozygous strain active dry yeast was kneaded with crushed ice at 0°C and flour, and the leavening time of the dough was determined. The heterozygous strain with the shortest leavening time of the corresponding dough was used as the target strain to screen out the heterozygous strain with cold permeation shock resistance. a high-sugar permeation pressure resistant, cold permeation shock resistant, and organic acid resistant heterozygous strain was obtained by screening, and the heterozygous strain was identified, the obtained colony of yeast strain was cheese-like in texture, milky white in color, smooth in surface, and neat in edge, oval in microscopic shape and budding.

This strain was named Saccharomyces cerevisiae AMCC 31195 strain. The Saccharomyces cerevisiae strain AMCC 31195 was deposited in the China Center for Type Culture Collection (CCTCC) on December 29, 2021, with deposit number CCTCC NO: M 20211685 (i.e. CCTCC M 20211685).

**Example II**

The Saccharomyces cerevisiae AMCC 31195 strain, the parent strain Saccharomyces cerevisiae AMCC 30002 strain, and Saccharomyces cerevisiae AMCC 30004 strain were inoculated into shake flasks containing YPD liquid medium respectively, shaken at 30°C, and cultured overnight. After centrifugation and washing, the yeast milk was obtained. The mass of the yeast milk, i.e. biomass, and water content was detected, and the dry weight and relative percentage were calculated. The results were shown in Table 3. The relative percentages were calculated as follows:

Dry weight (g/L) = Yeast milk mass * (1 - water content) Relative percentage = (heterozygous strain dry weight/parental strain AMCC30002 dry weight) * 100%

Table 3 Biomass dry weight (g/L) and relative percentage (%) of heterozygous strains

| Strain number | Dry weight | Relative percentage |
|---|---|---|
| AMCC 30002 | 13.54 | 100.0% |

(continued)

| Strain number | Dry weight | Relative percentage |
|---|---|---|
| AMCC 30004 | 13.52 | 99.9% |
| AMCC 31195 | 13.32 | 98.4% |

As shown in Table 3, the yeast Saccharomyces cerevisiae AMCC 31195 provided by the present invention was capable of reaching a biomass dry weight of 13.32 g/L after overnight culture in shake flasks.

**Example III**

The Saccharomyces cerevisiae AMCC 31195 strain and the parent strains Saccharomyces cerevisiae AMCC 30002 strain and Saccharomyces cerevisiae 30004 strain were detected for fermentation activity in Test A and Test B, Test A: 16% sugar dough system; Test B: 16% sugar + 1% calcium propionate dough system. The percentage of baking raw materials in the dough systems of Test A and Test B is detailed in Table 4 below. The weight of yeast milk required was calculated. Test A was detected for 1 h fermentation activity of 280 g of dough and Test B was detected for 3 h fermentation activity of 70 g of dough. The results were shown in Table 5. Among them, the calculation method of relative activity percentage in Table 5 is as follows:

Percentage of relative activity = (Fermentation activity of heterozygous strain/parent strain AMCC30002) * 100%

Table 4 Formulas of dough systems for Test A and Test B

| Test raw material | A (g) | B (g) |
|---|---|---|
| Flour | 100 | 100 |
| Sucrose | 16 | 16 |
| Salt | 1.4 | 1.4 |
| Dry yeast | 1 | 1 |
| Water | 45 | 45 |
| Calcium Propionate | - | 1 |

Table 5 Fermentation activity (mL) and relative activity percentage (%) of heterozygous strains in Test A and Test B

| Strain number | 16% sugar 1 h gas production | Percentage of relative activity | 16% sugar + 1% calcium propionate 3 h gas production | Percentage of relative activity |
|---|---|---|---|---|
| AMCC 30002 | 694 | 100.0 | 648 | 100.0 |
| AMCC 30004 | 705 | 101.6 | 609 | 94.0 |
| AMCC 31195 | 667 | 96.1 | 621 | 95.8 |

As shown in Table 5, the Saccharomyces cerevisiae AMCC 31195 strain provided by the present invention produced 667 ml of gas at 280 g of dough fermented for 1 h in a 16% sugar dough system and 621 ml of gas at 70 g of dough fermented for 3h in a 16% sugar + 1% calcium propionate dough system.

**Example IV**

The Saccharomyces cerevisiae AMCC 31195 strain and the parent strain Saccharomyces cerevisiae AMCC 30002 strain and Saccharomyces cerevisiae 30004 were inoculated into YPD liquid medium for culturing in a 30 L fermenter, respectively. After separation, washing, suction filtration, pressure filtration, pelleting, drying, and packaging, the active dry yeast was obtained. Then the fermentation activity of 280 g dough was detected in test C, and the detection time was 2 h. Test C: The baking raw material percentage of 16% sugar + 0.6% calcium propionate dough system was shown in Table 6 below, and the detection results were shown in Table 7. Wherein, the calculation method of the relative activity in Table 7 was as follows:

Relative activity = (Fermentation activity of heterozygous strain/Fermentation activity of parent strain AMCC 30002) * 100%

Table 6 Baking raw material formula for dough system in Test C

| Test raw material | C (g) |
| --- | --- |
| Flour | 100 |
| Sucrose | 16 |
| Salt | 1.4 |
| Dry yeast | 1 |
| Water | 45 |
| Calcium propionate | 0.6 |

Table 7 Fermentation activity (mL) and relative activity percentage (%) of heterozygous strains in Test C

| Strain number | 16% sugar + 0.6% calcium propionate 2 h gas production | Relative activity |
| --- | --- | --- |
| AMCC 30002 | 820 | 100.0 |
| AMCC 30004 | 763 | 93.0 |
| AMCC 31195 | 955 | 116.5 |

As shown in Table 7, the active dry yeast produced by the Saccharomyces cerevisiae strain AMCC 31195 provided by the present invention produced 955 ml of gas after fermentation of 280 g of dough in a 16% sugar + 0.6% calcium propionate dough system for 2 h.

**Example V**

Active dry yeast of Saccharomyces cerevisiae AMCC 31195 strain and active dry yeast of parent strain Saccharomyces cerevisiae AMCC 30002 strain and active dry yeast of Saccharomyces cerevisiae AMCC 30004 strain were used to prepare 400 g dough of the six application formulas shown in Table 8. The parent strain AMCC 30002 with more advantages in each trait was selected as the control strain. Taking the fermentation time as the index, the shorter the fermentation time indicated that the faster the dough started at the same volume, the yeast strain had more advantages in the traits. The percentage of baking raw materials for each application formula is shown in Table 8 below (the water for Formula 4 is 0°C crushed ice for the experiment); Formula 1: 15% sugar + 0.3% calcium propionate dough system; Formula 2: 20% sugar dough system; Formula 3: 20% sugar + 0.5% calcium propionate dough system; Formula 4: 20% sugar cold permeation shock dough system; Formula 5: 25% sugar + 0.6% calcium propionate dough system; Formula 6: 40% sugar + 20% butter dough system. The results were shown in Table 9. Wherein, the percentage of fermentation time relative to the control strain in Table 9 was calculated as follows:

Percentage of fermentation time relative to control strain = (fermentation time of heterozygous strains/fermentation time of parent strain AMCC30002) * 100%

Table 8 Baking raw material formula of application formula

| Raw material | Formula 1 (g) | Formula 2 (g) | Formula 3 (g) | Formula 4 (g) | Formula 5 (g) | Formula 6 (g) |
| --- | --- | --- | --- | --- | --- | --- |
| Flour | 100 | 100 | 100 | 100 | 100 | 100 |
| Yeast | 1 | 1 | 1 | 1 | 1 | 1.2 |
| Common salt | 1 | 1 | 1 | 1 | 1 | 1.5 |
| Butter | 8 | 8 | 8 | 8 | 8 | 20 |
| Sucrose | 15 | 20 | 20 | 20 | 25 | 40 |
| Calcium propionate | 0.3 | 0 | 0.5 | / | 0.6 | / |
| Egg | / | / | / | / | / | 5 |
| Water | 62 | 56 | 56 | 56 (ice) | 54 | 50 |

Table 9 Fermentation time and relative percentage (%) of application test

| formulas | AMCC 30002 strain fermentation time (min) | AMCC 31195 strain fermentation time (min) | Percentage of fermentation time relative to the control strain |
|---|---|---|---|
| Formula 1 | 121.8 | 116.6 | 95.7 |
| Formula 2 | 121.3 | 120.7 | 99.5 |
| Formula 3 | 150.5 | 148.2 | 98.5 |
| Formula 4 | 161.7 | 152.5 | 94.3 |
| Formula 5 | 178.3 | 170.4 | 95.6 |
| Formula 6 | 175.3 | 167.1 | 95.3 |

As shown by the results in Table 9, the saccharomyces cerevisiae AMCC 31195 strain had advantages in all the six application formulas, indicating that the strain could grow normally in a 15-40% sugar dough system and had high-sugar permeation pressure resistance; The advantages of Formula 1, Formula 3 and Formula 5 showed that their organic acid resistance was stronger than that of AMCC 30002 strain. In Formula 4, the advantage was the most obvious, the fermentation time was 5.7% faster than that of the AMCC 30002 strain, indicating that it had cold permeation shock resistance. In Formula 6, the fermentation time was 4.7% faster than that of the AMCC 30002 strain, indicating that it was resistant in a heavy oil and heavy sugar system and could meet the application needs of heavy oil and heavy sugar products.

The foregoing is merely a preferred embodiment of the invention and is not to be construed as limiting the invention. Thus, it is intended that the present invention cover the modifications and variations of this invention provided they come within the scope of the appended claims and their equivalents.

## Claims

1. A Saccharomyces cerevisiae strain, wherein the Saccharomyces cerevisiae strain is:
Saccharomyces cerevisiae AMCC 31195 strain, which is deposited in the China Center for Type Culture Collection (CCTCC) with deposit number CCTCC NO: M 20211685.

2. A fermentation preparation method for a Saccharomyces cerevisiae microbial inoculum, comprising the step of culturing the Saccharomyces cerevisiae strain according to claim 1.

3. The preparation method according to claim 2, wherein the preparation method comprises the steps of:

    (1) amplifying and culturing the Saccharomyces cerevisiae strain according to claim 1;
    (2) adding the product obtained in step (1) to a liquid medium, and fermenting and culturing at 26-32°C.

4. A microbial inoculum, comprising the Saccharomyces cerevisiae AMCC 31195 strain according to claim 1.

5. The microbial inoculum according to claim 4, wherein the microbial inoculum is obtained by the fermentation preparation method according to claim 3 or 4.

6. Use of the Saccharomyces cerevisiae strain according to claim 1 or the microbial inoculum according to claim 4 or 5 for fermentation.

7. Use of the Saccharomyces cerevisiae strain according to claim 1 or the microbial inoculum according to claim 4 or 5 in a dough.

8. A dough, comprising the Saccharomyces cerevisiae strain according to claim 1 or the microbial inoculum according to claim 4 or 5.

9. The dough according to claim 8, wherein the dough comprises flour and the Saccharomyces cerevisiae strain in a mass ratio of 100:0.5-5.

10. The dough according to claim 8 or 9, wherein the dough comprises flour and sugar in a mass ratio of 100:0-40;

preferably, the dough comprises flour and sugar in a mass ratio of 100:15-40;
preferably, the sugar is sucrose.

11. The dough according to any one of claims 8 to 10, wherein the dough further comprises a preservative;

    preferably, the preservative is an organic acid and salts thereof;
    preferably, the organic acid is one or two of propionic acid and acetic acid, and the organic acid salt is one or a combination of two or more of calcium propionate, sodium propionate, and sodium acetate;
    preferably, the mass ratio of flour to preservative in the dough is 100:0-1, preferably 100:0-0.6.

12. The preparation method for a dough according to any one of claims 8 to 11, wherein the preparation method comprises the steps of kneading the dough with water at 0-35°C.

13. A dough product, preparing from the dough according to any one of claims 8 to 11.

14. The dough product according to claim 13, wherein the dough product is bread.

| INTERNATIONAL SEARCH REPORT | International application No. |
|---|---|
| | **PCT/CN2023/079575** |

**A. CLASSIFICATION OF SUBJECT MATTER**

C12N1/18(2006.01)i;  A21D8/04(2006.01)i;  C12R1/865(2006.01)n

According to International Patent Classification (IPC) or to both national classification and IPC

**B. FIELDS SEARCHED**

Minimum documentation searched (classification system followed by classification symbols)

IPC:C12N,A21D,C12R

Documentation searched other than minimum documentation to the extent that such documents are included in the fields searched

Electronic data base consulted during the international search (name of data base and, where practicable, search terms used)

VCN, VEN, WPABSC, ENTXT, OETXT, CNKI, PubMed, Elsevier Science, ISI WEB of Science: 耐冷, 耐冷渗透, 渗透休克, 酿酒酵母, 丙酸钙, AMCC31195, AMCC30002, AMCC30004, CTCC s 20211685, Saccharomyces cerevisiae, cold resist+, cold shock, dough develop+, Calcium propionate

**C. DOCUMENTS CONSIDERED TO BE RELEVANT**

| Category* | Citation of document, with indication, where appropriate, of the relevant passages | Relevant to claim No. |
|---|---|---|
| A | CN 113355251 A (ANGEL YEAST CO., LTD.) 07 September 2021 (2021-09-07)<br>see abstract, and embodiments 3-4 | 1-14 |
| A | JP H0779767 A (KANEGAFUCHI CHEMICAL INDUSTRY CO., LTD.) 28 March 1995 (1995-03-28)<br>see abstract | 1-14 |
| A | JP H08154666 A (KANEGAFUCHI CHEMICAL INDUSTRY CO., LTD.) 18 June 1996 (1996-06-18)<br>see abstract | 1-14 |
| A | US 2014010919 A1 (LESAFFRE ET COMPAGNIE) 09 January 2014 (2014-01-09)<br>see abstract, and embodiments 3.1-3.2 | 1-14 |
| A | 刘文玉 (LIU, Wenyu). "酿酒酵母低温耐受机制的研究进展 (Advances in Research on the Mechanism of Low Temperature Tolerance of Saccharomyces Cerevisiae)"<br>酿酒 (Liquor Making), Vol. 44, No. 5, 30 September 2017 (2017-09-30),<br>see pp. 11-12 | 1-14 |

☐ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **27 June 2023** | **06 July 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

INTERNATIONAL SEARCH REPORT

Information on patent family members

International application No.

**PCT/CN2023/079575**

| Patent document cited in search report | | | Publication date (day/month/year) | Patent family member(s) | | | Publication date (day/month/year) |
|---|---|---|---|---|---|---|---|
| CN | 113355251 | A | 07 September 2021 | None | | | |
| JP | H0779767 | A | 28 March 1995 | None | | | |
| JP | H08154666 | A | 18 June 1996 | JP | 3766701 | B2 | 19 April 2006 |
| US | 2014010919 | A1 | 09 January 2014 | CR | 20130385 | A | 29 November 2013 |
| | | | | CO | 6771431 | A2 | 15 October 2013 |
| | | | | AP | 3795 | A | 31 August 2016 |
| | | | | CA | 2827164 | A1 | 23 August 2012 |
| | | | | CA | 2827164 | C | 29 October 2019 |
| | | | | CL | 2013002374 | A1 | 28 March 2014 |
| | | | | GT | 201300200 | A | 14 April 2016 |
| | | | | FR | 2971790 | A1 | 24 August 2012 |
| | | | | FR | 2971790 | B1 | 23 June 2017 |
| | | | | PE | 20140733 | A1 | 10 July 2014 |
| | | | | PL | 2683248 | T3 | 31 July 2018 |
| | | | | BR | 112013020518 | A2 | 18 October 2016 |
| | | | | BR | 112013020518 | B1 | 14 May 2019 |
| | | | | ECSP | 13012842 | A | 31 October 2013 |
| | | | | ZA | 201306036 | B | 29 October 2014 |
| | | | | TR | 201806700 | T4 | 21 June 2018 |
| | | | | EP | 2683248 | A1 | 15 January 2014 |
| | | | | EP | 2683248 | B1 | 28 February 2018 |
| | | | | KR | 20140030133 | A | 11 March 2014 |
| | | | | KR | 101879165 | B1 | 17 July 2018 |
| | | | | ES | 2669569 | T3 | 28 May 2018 |
| | | | | UA | 111839 | C2 | 24 June 2016 |
| | | | | JP | 2014505487 | A | 06 March 2014 |
| | | | | JP | 5898237 | B2 | 06 April 2016 |
| | | | | NI | 201300069 | A | 13 February 2014 |
| | | | | US | 9138006 | B2 | 22 September 2015 |
| | | | | MY | 162342 | A | 15 June 2017 |
| | | | | SG | 192811 | A1 | 30 September 2013 |
| | | | | AR | 082082 | A1 | 07 November 2012 |
| | | | | DOP | 2013000183 | A | 15 December 2013 |
| | | | | AU | 2011359063 | A1 | 05 September 2013 |
| | | | | AU | 2011359063 | B2 | 29 January 2015 |
| | | | | RU | 2013142444 | A | 27 March 2015 |
| | | | | RU | 2571934 | C2 | 27 December 2015 |
| | | | | WO | 2012110711 | A1 | 23 August 2012 |
| | | | | MX | 2013009321 | A | 01 October 2013 |
| | | | | MX | 345223 | B | 20 January 2017 |

Form PCT/ISA/210 (patent family annex) (July 2022)